# EUROPÄISCHE PATENTSCHRIFT

(11) **EP 0 696 568 B1**
(45) Veröffentlichungstag und Bekanntmachung des Hinweises auf die Patenterteilung: **10.05.2000**
(21) Anmeldenummer: 95110193.0
(22) Anmeldetag: 30.06.1995
(51) Int. Cl.: C07C 69/738, C07C 67/343, C07D 453/02

(54) **Verfahren zur Herstellung von substituierten 4-Methyliden-Zimtsäurederivaten sowie neue Methylidenzimtsäurederivate**
Process for preparing substituted 4-methylidene cinnamic acid derivatives as well as novel methylidene cinnamic acid derivatives
Procédé pour la préparation des dérivés de l'acide 4-méthylidene cinnamique ainsi que des nouveaux dérivés de l'acide 4-méthylidene cinnamique

(30) Priorität: 12.07.1994 DE 4424489
(43) Veröffentlichungstag der Anmeldung: 14.02.1996
(73) Patentinhaber: MERCK PATENT GmbH, 64293 Darmstadt (DE)
(72) Erfinder: Poetsch, Eike, Dr., D-64367 Mühltal (DE); Meyer, Volker, D-64846 Gross-Zimmern (DE); Heywang, Ulrich, Dr., D-64289 Darmstadt (DE); Stein, Inge, Dr., D-64390 Erzhausen (DE); Schwarz, Michael, Dr., D-64521 Gross-Gerau (DE); Kompter, Michael, Dr., D-64560 Riedstadt (DE)

(56) Entgegenhaltungen:
- EP-A- 0 078 768
- EP-A- 0 103 544
- DE-A- 3 445 365
- US-A- 3 560 510
- J. MED. CHEM., Bd. 18, Nr. 1, 1975 Seiten 71-74, WARAWA 'Quinuclidine Chemistry. 1.'
- J. MED. CHEM., Bd. 17, Nr. 5, 1974 Seiten 497-501, E. J. WARA ET AL. 'Quinuclidine Chemistry. 2.'
- CHRISTEN , VÖGTLE 'Organische Chemie II' 1990 , OTTO SALLE VERLAG , FRANKFURT * Seite 342 - Seite 344 *

## Beschreibung

Die vorliegende Erfindung betrifft ein Verfahren zur Herstellung von substituierten 4-Methyliden-zimtsäurederivaten der Formel (I) 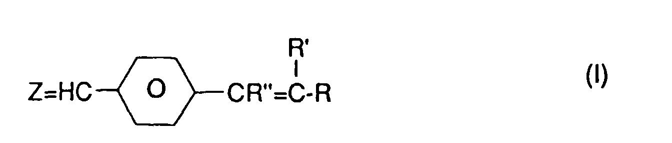 worin
- R: für CN oder COR¹ steht, wobei R¹ OH, O-C₆-C₁₀-Aryl, O-C₁-C₂₀-Alkyl, NH₂, NH-C₆-C₁₀-Aryl, NH-C₁-C₂₀-Alkyl, N-C₁-C₂₀-Alkyl-C₆-C₁₀-Aryl oder N-di-C₁-C₂₀-Alkyl bedeutet,
- R': für H, C₁-C₂₀-Alkyl oder R steht,
- R": für H, C₁-C₂₀-Alkyl, C₆-C₂₀-Aryl oder einem Rest der Formel 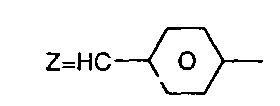 steht, und
- Z =: für einen Rest ausgewählt aus den Formeln II, III und IV 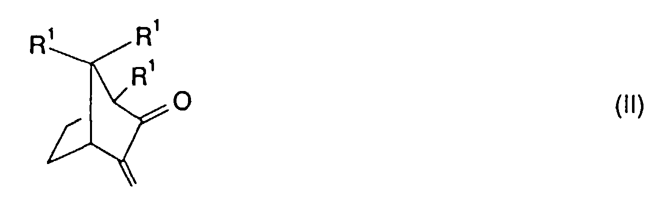 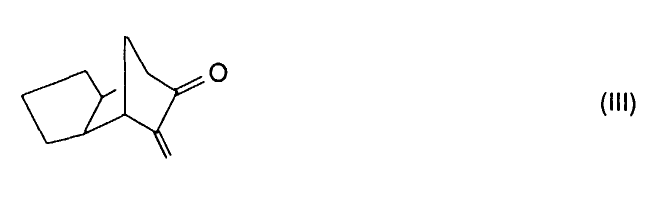 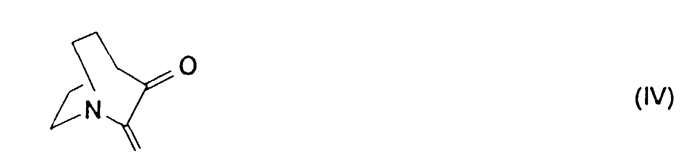 worin R¹ H oder Methyl bedeutet,
steht,
dadurch gekennzeichnet, daß man ein Arylhalogenid der Formel (V), 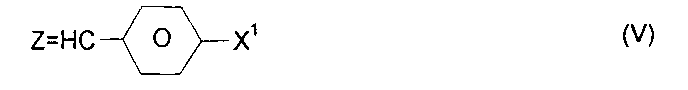 worin Z die angegebene Bedeutung besitzt, und
- X¹: Cl, Br oder Jod bedeutet, mit einem Acrylsäure-Derivat der Formel (VI), 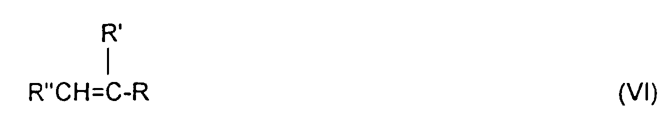 worin R, R' und R" die angegebene Bedeutung besitzen, in Gegenwart von Palladium-Katalysatoren umsetzt.

Es ist bereits bekannt, daß Olefine in Gegenwart von Palladium-Katalysatoren, Phosphanliganden und einer Base durch Bromaromaten aryliert werden können (siehe R.F. Heck, Org. React. 27, 345-391 (1982)).

Weiterhin werden z.B. in der EP 0 078 768 und der EP 0 103 544 Heck-Kopplungen zwischen 4-substituierten Arylhalogeniden und Acrylsäure-Derivaten beschrieben. Es findet sich dort jedoch kein Hinweis, daß dieses Verfahren auch bei Arylhalogeniden angewandt werden kann, welche in 4-Stellung zum Halogenatom eine möglicherweise zu einer Heck-Kopplung befähigte Alkenylgruppe aufweisen.

In der DE 34 45 365 wird die Herstellung der Verbindungen der Formel worin Z eine Gruppe der Formel II bedeutet, in einem aufwendigen 4-stufigen Verfahren ausgehend von 4'-Ethylbenzylidencampher beschrie ben, welches zudem zwei Wittig-Reaktionsstufen umfaßt.

Es besteht deshalb das Bedürfnis nach einem Verfahren, mit dem die Verbindungen der Formel I mit weniger Verfahrensschritten, in hohen Raum-Zeit-Ausbeuten und unter Verwendung von keinen besonderen Aufwand erfordernden Hilfsmitteln hergestellt werden können.

Es wurde nun ein Verfahren zur Herstellung von substituierten 4-Methyliden-zimtsäurederivaten der Formel (I) 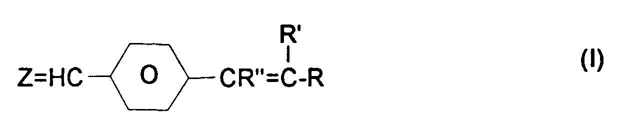 worin
- R: für CN oder COR¹ steht, wobei R¹ OH, O-C₆-C₁₀-Aryl, O-C₁-C₂₀-Alkyl, NH₂, NH-C₆-C₁₀-Aryl, NH-C₁-C₂₀-Alkyl, N-C₁-C₂₀-Alkyl-C₆-C₁₀-Aryl oder N-di-C₁-C₂₀-Alkyl bedeutet,
- R': für H, C₁-C₂₀-Alkyl oder R steht
- R": für H, C₁-C₂₀-Alkyl, C₆-C₂₀-Aryl oder einem Rest der Formel 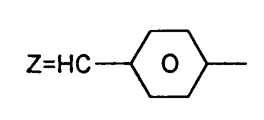 steht, und
- Z =: für einen Rest ausgewählt aus den Formeln II, III und IV 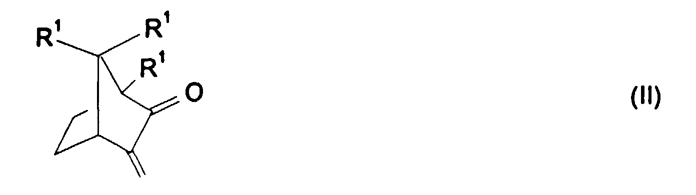 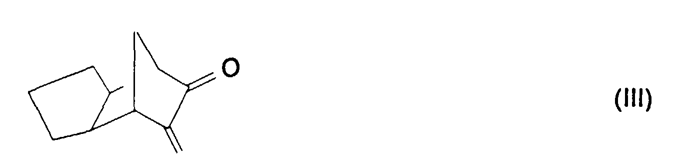 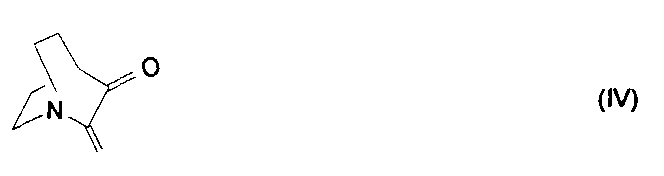 worin R¹ H oder Methyl bedeutet,
steht,
welches dadurch gekennzeichnet ist, daß man ein Arylhalogenid der Formel (V) 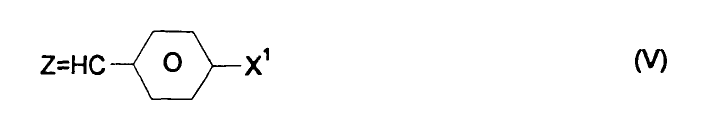 worin Z die angegebene Bedeutung besitzt, und
- X¹: Cl, Br oder Jod bedeutet, mit einem Acrylsäure-Derivat der Formel (VI), 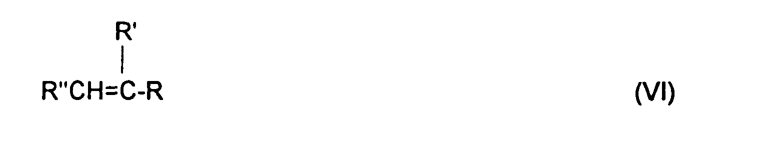 worin R, R' und R" die angegebene Bedeutung besitzen in Gegenwart von Palladium-Katalysatoren umsetzt.

Bevorzugte Ausführungsformen sind:
a) Verfahren, wobei man als Palladium-Katalysatoren homogene Palladium-Katalysatoren der Oxidationsstufe 0 und/oder +2 in Mengen von 0,0001 bis 1 Mol-% Palladium bezogen auf eingesetztes Arylhalogenid der Formel (V) einsetzt.
b) Verfahren, wobei man ein Molverhältnis von Arylhalogenid der Formel (V) zu Acrylsäure-Derivat der Formel (VI) im Bereich von 1 : 0,7 bis 1 : 3 einhält.
c) Verfahren, wobei man das Arylhalogenid der Formel (V) herstellt in dem man eine dem Arylhalogenid der Formel (V) entsprechende Verbindung, worin Z O bedeutet, mit Campher, Norcampher, 8-Ketotricyclo[5.2.1.0^{2,6}]deca n oder Chinuclidinon in Gegenwart einer Base oder einer Lewis-Säure umsetzt.

Die Verbindungen der Formel I sind teilweise bekannt (Z = Formel II) aus der DE 34 45 365 und teilweise neu.

Die Verbindungen der Formel V sind teilweise bekannt und teilweise neu. Die Verbindungen der Formel V, worin Z einen Rest der Formel lll bedeutet, sind ebenfalls Gegenstand der Erfindung.

Die Bedeutungen der Reste R, R' und R" sind für die Durchführung des erfindungsgemäßen Verfahrens unkritisch. In den Verbindungen der Formeln I und VI bedeutet R vorzugsweise CN oder CO-O-C₁-C₂₀-Alkyl, insbesondere CO-O-C₁-C₁₀-Alkyl.

In den Verbindungen der Formeln I und VI bedeutet R' vorzugsweise H oder C₁-C₆-Alkyl oder weist eine der bevorzugten Bedeutungen von R auf. Insbesondere bevorzugt ist R' H oder CH₃.

In den Verbindungen der Formeln I und VI bedeutet R'' vorzugsweise H, C₁-C₆-Alkyl oder C₆-C₁₀-Aryl.

Der Begriff C₆-C₂₀-Aryl umfaßt homo- und heteroaromatische Reste, insbesondere unsubstituierte und substituierte Phenyl- oder Naphthalinreste, worin auch eine oder mehrere CH-Gruppen durch N ersetzt sein können.

Das Molverhältnis von Arylhalogenid zu Acrylsäurederivat kann beliebig gewählt werden. Vorzugsweise arbeitet man bei einem Molverhältnis von Chloraromat zu Acrylsäurederivat im Bereich von 1 : 0,7 bis 1 : 3. Besonders bevorzugt beträgt dieses Verhältnis 1 : 0,8 bis 1 : 1,5.

In einem bevorzugten Verfahren zur Herstellung der Verbindungen der Formel I, worin R'' für einen Rest der Formel 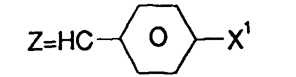 steht, wird das Arylhalogenid der Formel V mit einem Acrylsäure-Derivat der Formel VI worin R'' für H steht, in einem Molverhältnis von 1 : 0,3 bis 1 : 0,6 umgesetzt.

Für das erfindungsgemäße Verfahren kommen als anorganische Basen beispielsweise Alkali- und Erdalkalisalze schwacher Säuren, vorzugsweise Alkali- und Erdalkalihydrogencarbonate und/oder -carbonate in Frage. Besonders bevorzugt wird Natriumcarbonat verwendet. Das Verhältnis von Chloraromat zu Base wird vorzugsweise so gewählt, daß pro Mol Arylhalogenid 0,3 bis 2, besonders bevorzugt 0,4 bis 1,3 Äquivalente Base eingesetzt werden.

Die bevorzugten homogenen Palladium-Katalysatoren weisen in der Regel Phosphinliganden auf. Phosphinliganden sind kommerziell erhältlich oder können in Analogie zu an sich bekannten Verfahren hergestellt werden.

In den bevorzugten monodentaten Phosphinliganden der Formel P (R²)₃ sind die Reste vorzugsweise gleich und weisen eine verzweigte Alkyl- oder Alkoxygruppe bzw. eine Cyclohexylgruppe oder eine Aryl, insbesondere Phenyl- oder o-Tolylgruppe auf.

Bevorzugte monodentate Phosphinliganden sind demnach:
Trimethylphosphin, Triethylphosphin, Tripropylphosphin, Triisopropylphosphin, Tributylphosphin, Tricyclohexylphosphin, Trimethylphosphit, Trimethylphosphit, Tripropylphosphit, Triisopropylphosphit, Tributylphosphit und Tricyclohexylphosphit, insbesondere Triphenylphosphin, Tri(o-tolyol)phosphin, Triisopropylphosphin oder Tricyclohexylphosphin.

In den bevorzugten bidentaten Phosphinliganden (R²)₂P-(R³)-P(R²)₂ sind die Reste R² jeweils gleich und R³ vorzugsweise divalentes C₂₋₆-Alkylen bedeutet.

Bevorzugte bidentate Phosphinliganden sind demnach:
1,2-Bis(dimethylphosphin)ethan, 1,2-Bis(diethylphosphin)-ethan, 1,2-Bis(dipropylphosphin)ethan, 1,2-Bis(diisopropylphosphin)ethan, 1,2-Bis(dibutylphosphin)ethan, 1,2-Bis(dicyclohexylphosphin)ethan, 1,3-Bis(dicyclohexylphosphin)propan, 1,3-Bis(diisopropylphosphin)propan, 1,4-Bis-(diisopropyl-phosphin)-butan und 2,4-Bis(dicyclohexylphosphin)-pentan.

Phosphane können in das erfindungsgemäße Verfahren beispielsweise in einem Mol-Verhältnis von Palladium: Phosphor von 1 : 0,8 bis 1 : 2,5 eingesetzt werden. Bevorzugt beträgt dieses Verhältnis 1 : 0,9 bis 1 : 2,3, besonders bevorzugt etwa 1 : 2.

Es versteht sich von selbst, daß sich die angegebenen Verhältnisse auf die monodentaten Phosphane beziehen, bei den bidentaten Phosphanen werden in der Regel Palladium: Phosphan-Verhältnisse von 1 0,4 bis 1 : 1,25 eingesetzt.

In einer besonders bevorzugten Ausführungsform können auch homogene Palladiumkatalysatoren eingesetzt werden, welche gemischte Liganden aufweisen, d.h. sowohl mono- als auch bidentate Phosphine.

Das erfindungsgemäße Verfahren kann man beispielsweise bei Temperaturen im Bereich von 50 bis 180 °C durchführen. Bevorzugte Temperaturen liegen im Bereich von 80 bis 150 °C, besonders bevorzugt im Bereich des Siedepunktes des eingesetzten Lösungsmittels. Das erfindungsgemäße Verfahren wird üblicherweise bei Normaldruck durchgeführt. Es kann jedoch auch bei vermindertem oder erhöhtem Druck durchgeführt werden. Die Anwendung von erhöhtem Druck ist insbesondere dann angezeigt, wenn man bei einer Reaktionstemperatur arbeiten möchte, bei der einzelne Bestandteile des Reaktionsgemisches bei Normaldruck sieden.

Das erfindungsgemäße Verfahren wird im allgemeinen unter einem Schutzgas, z.B. Stickstoff, und unter Rühren durchgeführt.

Geeignete Lösungsmittel sind Kohlenwasserstoffe, wie zum Beispiel Toluol, Ether, wie Tetrahydrofuran, polare, aprotische Lösemittel, wie zum Beispiel N-Methyl-pyrrolidon, Dimethylformamid, N,N-Dimethylacetonitril oder Dimethylsulfoxid.

Nach Durchführung des erfindungsgemäßen Verfahrens können die entstandenen anorganischen Salze beispielsweise durch einfaches Abfiltrieren oder Abnutschen abgetrennt werden. Man kann die Salze auch absitzen lassen und die restliche Reaktionsmischung abdekantieren.

Eine mögliche Ausführungsform des erfindungsgemäßen Verfahrens wird im folgenden beispielhaft an der Umsetzung von 4'-Chlorbenzylidencampher mit Acrylsäure-2-ethylhexylester erläutert:
4'-Chlorbenzylidencampher, Acrylsäure-2-ethylhexylester, Natriumcarbonat und N-Methylpyrrolidon (NMP) werden vorgelegt. Trialkylphosphan und Palladium(II)chlorid werden in einem Mol-Verhältnis von Palladium : Phosphor von 1 : 2 zugesetzt. Die Reihenfolge der Zugabe dieser Komponenten kann beliebig verändert werden. Dann wird das Gemisch unter Stickstoff und kräftigem Rühren auf 140 bis 150 °C erhitzt. Wenn festgestellt worden ist, daß sich kein 4'-Chlorbenzylidencampher mehr umsetzt, wird die Reaktion abgebrochen, das heißt, auf Raumtemperatur abgekühlt und auf Wasser gegossen. Dann wird die wäßrige Phase abgetrennt, mit einem organischen Lösungsmittel (z.B. Methyl-tert.-Butylether (MTB) extrahiert. Die erhaltenen vereinigten Extrakte werden vom Lösungsmittel befreit und im Vakuum destilliert.

In einer besonders bevorzugten Ausführungsform wird das erfindungsgemäße Verfahren in Gegenwart von Phasentransferkatalysatoren durchgeführt, z.B. von Tetraakylammonium-Salzen, insbesondere Tetrabutylammoniumchlorid (T. Jeffery, J. Chem. Soc., Chem. Commun. 1287-1289 1984).

Es ist überraschend, daß die erfindungsgemäße palladiumkatalysierte Umsetzung von 4-Methylidenhalogenbenzolen, welche zur Heck-Kopplung befähigte olefinische Gruppen aufweisen, in einer Heck-Kopplung mit Acrylsäure-Derivaten umgesetzt werden können, ohne daß es zu ausbeutenmindernden Nebenreaktionen kommt. Ein weiterer Vorteil ist, daß man die Verbindung der Formel I in hohen Raum-Zeit-Ausbeuten herstellen kann.

Das erfindungsgemäße Verfahren ermöglicht die Herstellung von Verbindungen der Formel I unter vorteilhaften Reaktionsbedingungen, wobei kein besonderer Aufwand für die Handhabung von Hilfsmitteln (Basen, Lösungsmittel) nötig ist. Die benötigten Basen, Lösungsmittel und Phosphane sind leicht zugänglich und preisgünstig.

Das erfindungsgemäße Verfahren ist wesentlich wirtschaftlicher und erreicht im Vergleich zu den Verfahren des Standes der Technik und eine höhere Raum-Zeit-Ausbeute.

Die Verbindungen der Formel I können beispielsweise als UV-Absorber in Kosmetika eingesetzt werden (siehe DE 34 35 365).

### Beispiel 1

### Herstellung einer Verbindung der Formel I, worin

Z = Formel II, R¹ = CH₃, R' = H und R = -CO-O-CH₂-CH(C₂H₅)-C₄H₉ 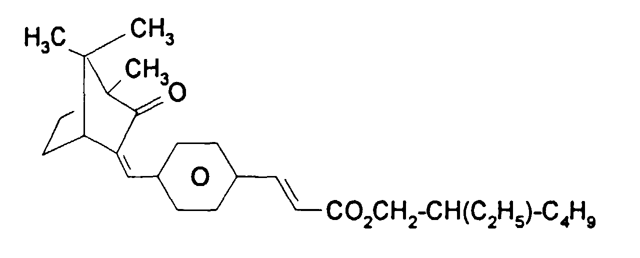 In einem Vierhalsrundkolben wurden 54,8 g 4'-Chlorbenzylidencampher, 52,3 g Acrylsäure-2-ethylhexylester, 10,6 g Natriumcarbonat und 200 ml NMP vorgelegt und mit 2,3 mg Tricyclohexylphosphan und 0,9 mg Palladiumacetat (2,0 mol-% bezogen auf 4'-Chlorbenzylidencampher) versetzt. Das Gemisch wurde unter Stickstoff und Rühren auf 140-150 °C erhitzt. Nach 60 Stunden betrug der Umsatz gemäß Gaschromatographie 68 %, bezogen auf den eingesetzten 4'-Chlorbenzylidencampher.

Das Gemisch wird abgekühlt und auf Wasser gegossen. Nach Extraktion mit 3 × 200 ml MTB-Ether, wird die vereinigte organische Phase mit Wasser gewaschen, mit Natriumsulfat getrocknet und filtriert. Nach Entfernung des Lösungsmittels wird unter reduziertem Druck destilliert. Es ergibt sich eine Ausbeute an isoliertem Produkt von 48 g.

Analog werden hergestellt:

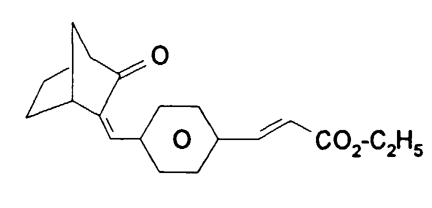

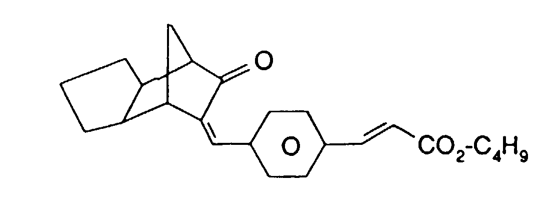

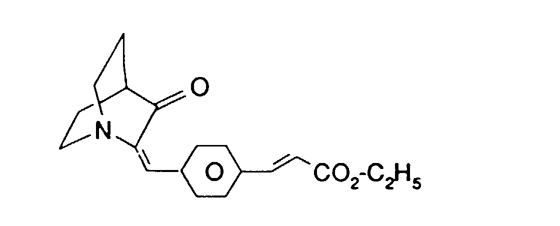

### Beispiel 2

Es wurde verfahren wie in Beispiel 1, jedoch wurde statt Tricyclohexylphosphan 1,5 mg Triisopropylphosphan zugesetzt. Nach 60 Stunden betrug der Umsatz gemäß Gaschromatographie 65 %, bezogen auf eingesetztes 4'-Chlorbenzylidencampher.

### Beispiel 3

Es wurde verfahren wie in Beispiel 1, jedoch wurde anstatt Tricyclohexylphosphan Triphenylphosphan und anstelle von 4'-Chlorbenzylidencampher 4'-Brombenzylidencampher zugesetzt. Nach 60 Stunden betrug der Umsatz gemäß Gaschromatographie 89 % bezogen auf eingesetztes 4'-Brombenzylidencampher.

### Beispiel 4

Es wurde verfahren wie in Beisiel 1, jedoch wurde die doppelte Menge an 4'-Chlorbenzylidencampher bezogen auf Acrylsäure-2-ethylhexylsäureester eingesetzt.

Man erhält die Verbindung der Formel

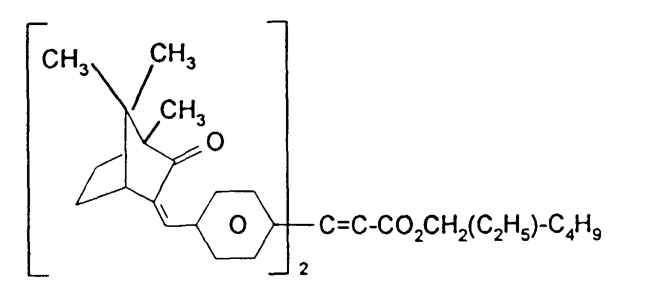

## Patentansprüche

1. Verfahren zur Herstellung von substituierten 4-Methylidenzimtsäurederivaten der Formel (I), 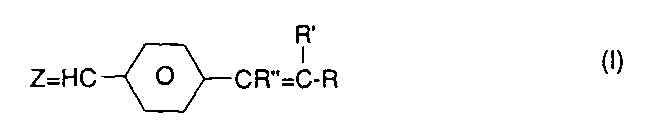 worin
R für CN oder COR¹ steht, wobei R¹ OH, O-C₆-C₁₀-Aryl, O-C₁-C₂₀-Alkyl, NH₂, NH-C₆-C₁₀-Aryl, NH-C₁-C₂₀-Alkyl, N-C₁-C₂₀-Alkyl-C₆-C₁₀-Aryl oder N-di-C₁-C₂₀-Alkyl bedeutet,
R' für H, C₁-C₂₀-Alkyl oder R steht,
R" für H, C₁-C₂₀-Alkyl, C₆-C₂₀-Aryl oder einem Rest der Formel 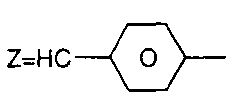 steht, und
Z = für einen Rest ausgewählt aus den Formeln II, III und IV 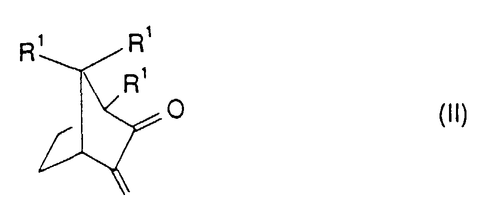 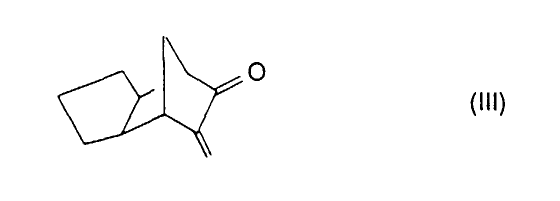 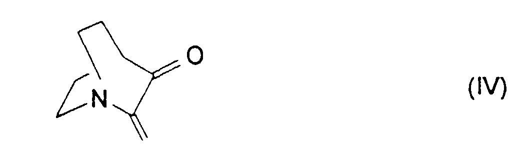 worin R¹ H oder Methyl bedeutet, steht,
dadurch gekennzeichnet, daß man ein Arylhalogenid der Formel (V), 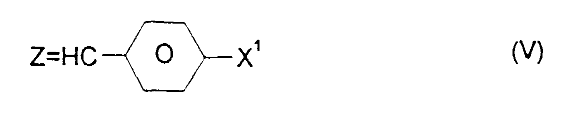 worin Z die angegebene Bedeutung besitzt, und
X¹ Cl, Br oder Jod bedeutet, mit einem Acrylsäure-Derivat der Formel (VI), 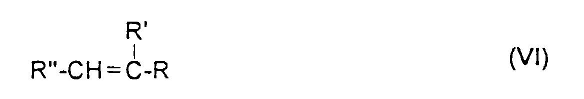 worin R, R' und R" die angegebene Bedeutung besitzen in Gegenwart von Palladium-Katalysatoren umsetzt.

2. Verfahren nach Anspruch 1, dadurch gekennzeichnet, daß man als Palladium-Katalysatoren homogene Palladium-Katalysatoren der Oxidationsstufe 0 und/oder +2 in Mengen von 0,0001 bis 1 Mol-% Palladium bezogen auf eingesetztes Arylhalogenid der Formel (V) einsetzt.

3. Verfahren nach Anspruch 1 oder 2, dadurch gekennzeichnet, daß man ein Molverhältnis von Arylhalogenid der Formel (V) zu Acrylsäure-Derivat der Formel (VI) im Bereich von 1 : 0,7 bis 1 : 3 einhält.

4. Verfahren nach einem der vorangehenden Ansprüche, dadurch gekennzeichnet, daß man das Arylhalogenid der Formel (V) herstellt in dem man eine dem Arylhalogenid der Formel (V) entsprechende Verbindung, worin Z O bedeutet, mit Campher, Norcampher, 8-Ketotricyclo[5.2.1.0^{2,6}]deca n oder Chinuclidinon in Gegenwart einer Base oder einer Lewis-Säure umsetzt.

5. Verfahren nach einem oder mehreren der vorhergehenden Ansprüche zur Herstellung von substituierten 4-Methylidenzimtsäurederivaten der Formel (I), 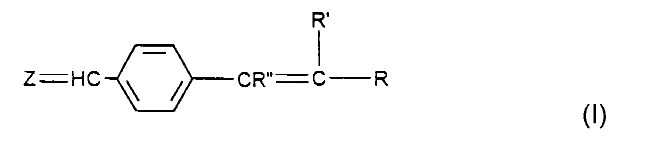 worin R, R' und R" die in Anspruch 1 gegebenen Bedeutungen haben, dadurch gekennzeichnet, daß Z für einen Rest ausgewählt aus den Formeln, III 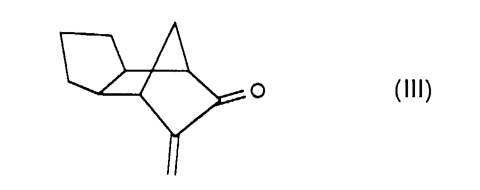 und IV 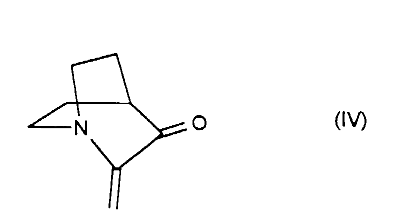 steht.

6. Arylhalogenid der Formel (V), 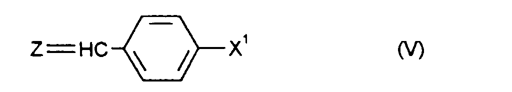 worin Z einen Rest der Formel III 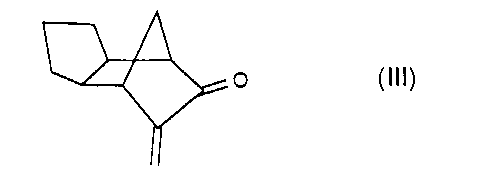 bedeutet und X¹ die in Anspruch 1 gegebenen Bedeutungen besitzt.

7. Verfahren zur Herstellung von substituierten 4-Methylidenzimtsäurederivaten der Formel (I), 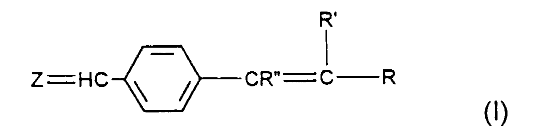 worin R, R' und Z die in Anspruch 1 gegebenen Bedeutungen haben, dadurch gekennzeichnet, daß R" für einen Rest der Formel 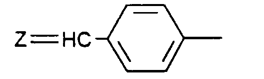 steht.

## Claims

1. Process for preparing substituted 4-methylidenecinnamic acid derivatives of the formula (I) 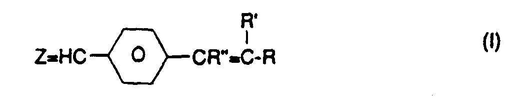 where
R is CN or COR¹, where R¹ is OH, O-C₆-C₁₀-aryl, O-C₁-C₂₀-alkyl, NH₂, NH-C₆-C₁₀-aryl, NH-C₁-C₂₀-alkyl, N-C₁-C₂₀-alkyl-C₆-C₁₀-aryl or N-di-C₁-C₂₀-alkyl,
R' is H, C₁-C₂₀-alkyl or R,
R" is H, C₁-C₂₀-alkyl, C₆-C₂₀-aryl or a radical of the formula 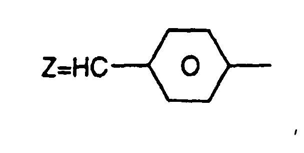 and
Z is a radical selected from among the formulae II, III and IV 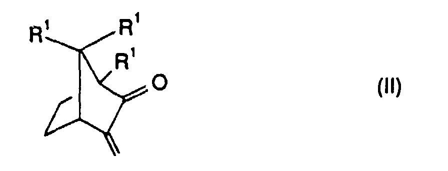 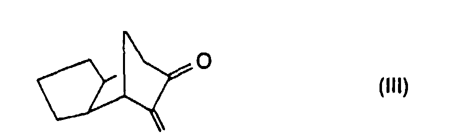 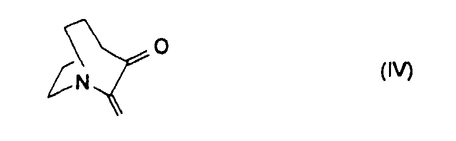 where R¹ is H or methyl,
characterized in that an aryl halide of the formula (V), 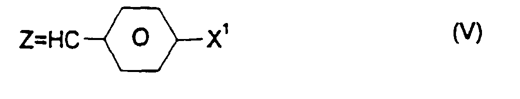 where Z is as defined above and
X¹ is Cl, Br or iodine, is reacted with an acrylic acid derivative of the formula (VI), 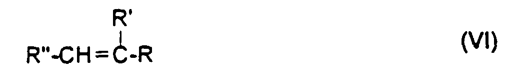 where R, R' and R" are as defined above, in the presence of palladium catalysts.

2. Process according to Claim 1, characterized in that the palladium catalysts used are homogeneous palladium catalysts of the oxidation state 0 and/or +2 in amounts of from 0.0001 to 1 mol % of palladium based on aryl halide of the formula (V) used.

3. Process according to Claim 1 or 2, characterized in that the molar ratio of aryl halide of the formula (V) to acrylic acid derivative of the formula (VI) is in the range from 1:0.7 to 1:3.

4. Process according to any one of the preceding claims, characterized in that the aryl halide of the formula (V) is prepared by reacting a compound which corresponds to the aryl halide of the formula (V) but in which Z is O with camphor, norcamphor, 8-ketotricyclo[5.2.1.0^{2,6}]decane or quinuclidinone in the presence of a base or a Lewis acid.

5. Process according to one or more of the preceding claims for preparing substituted 4-methylidenecinnamic acid derivatives of the formula (I), 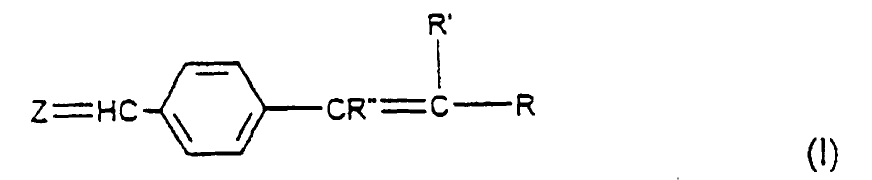 where R, R' and R" are as defined in Claim 1, characterized in that Z is a radical selected from among the formulae III 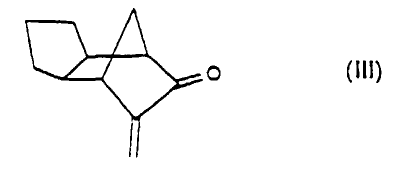 and IV

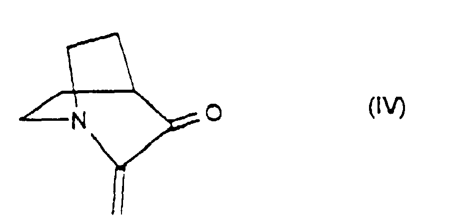

6. Aryl halide of the formula (V), 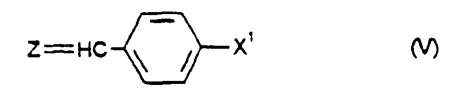 where Z is a radical of the formula III 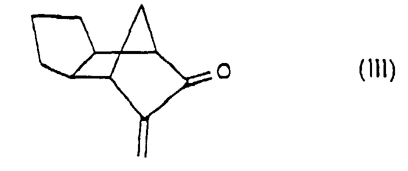 and X¹ is as defined in Claim 1.

7. Process for preparing substituted 4-methylidenecinnamic acid derivatives of the formula (I), 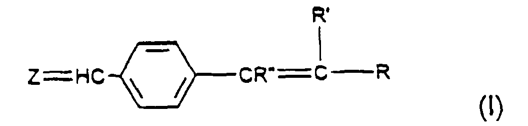 where R, R' and Z are as defined in Claim 1, characterized in that R" is a radical of the formula

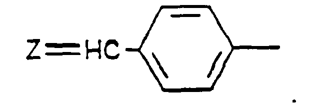

## Revendications

1. Procédé pour la préparation des dérivés de l'acide 4-méthylidènecinnamique substitués de formule (I) 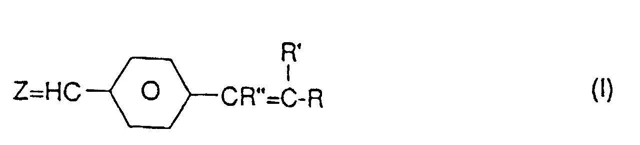 où
R représente CN ou COR¹, R¹ représentant OH, un O-aryle en C₆-C₁₀, un O-alkyle en C₁-C₂₀, NH₂, un NH-aryle en C₆-C₁₀, un NH-alkyle en C₁-C₂₀, un N-alkyle en C₁-₂₀ aryle en C₆-C₁₀ ou un N-dialkyle en C₁-C₂₀,
R' représente H, un alkyle en C₁-C₂₀ ou R,
R" représente H, un alkyle en C₁-C₂₀, un aryle en C₆-C₂₀ ou un reste de formule 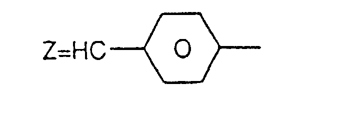 et
Z = un reste choisi parmi les formules II, III et IV 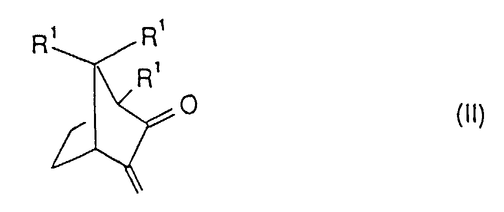 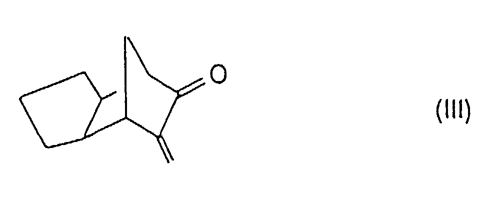 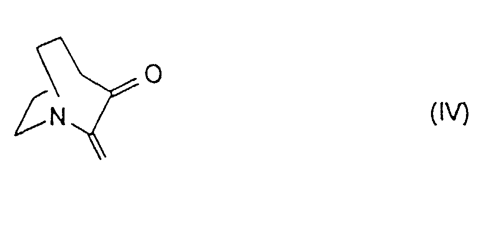
R¹ représentant H ou le méthyle,
caractérisé en ce que l'on fait réagir un halogénure d'aryle de formule V 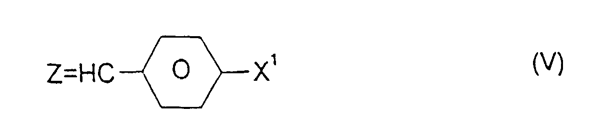 où Z a la signification indiquée et
X¹ représente Cl, Br ou l'iode,
avec un dérivé de l'acide acrylique de formule (VI) 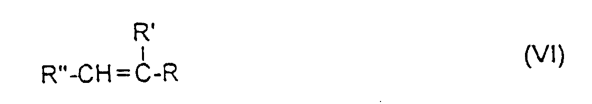 où R, R' et R" ont les significations indiquées, en présence de catalyseurs au palladium.

2. Procédé selon la revendication 1, caractérisé en ce que l'on utilise, comme catalyseurs au palladium, des catalyseurs au palladium homogènes de degré d'oxydation O et/ou +2 en quantités comprises entre 0,0001 et 1 % en mole rapporté à l'halogénure d'aryle de formule (V) utilisé.

3. Procédé selon la revendication 1 ou 2, caractérisé en ce que l'on maintient un rapport molaire de l'halogénure d'aryle de formule (V) au dérivé de l'acide acrylique de formule (VI) dans un intervalle compris entre 1:0,7 et 1:3.

4. Procédé selon l'une des revendications précédentes, caractérisé en ce que l'on prépare l'halogénure d'aryle de formule (V), en faisant réagir un composé correspondant à l'halogénure d'aryle de formule (V) où Z représente O, avec du camphre, du norcamphre, du 8-cétotricyclo[5.2.1.0^{2,6}] décane ou de la quinuclidinone en présence d'une base ou d'un acide de Lewis.

5. Procédé selon l'une ou plusieurs des revendications précédentes pour la préparation des dérivés de l'acide 4-méthylidènecinnamique substitués de formule (I) 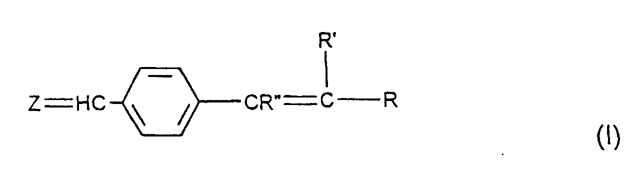 où R, R' et R" ont les significations données dans la revendication 1, caractérisé en ce que Z représente un reste choisi entre les formules III 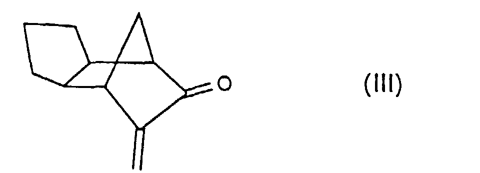 et IV

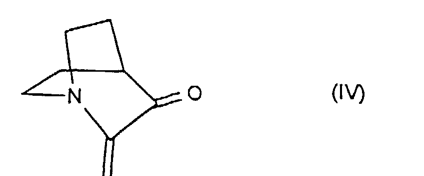

6. Halogénure d'aryle de formule (V) 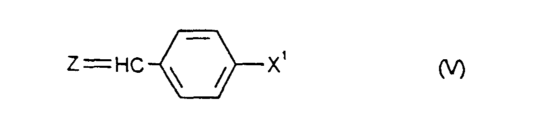 où Z représente un reste de formule III 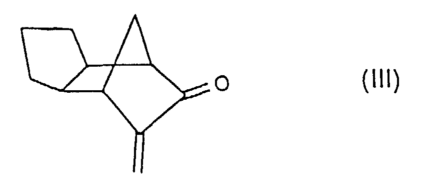 et X¹ a la signification donnée dans la revendication 1.

7. Procédé pour la préparation des dérivés de l'acide 4-méthylidènecinnamique substitués de formule (I) 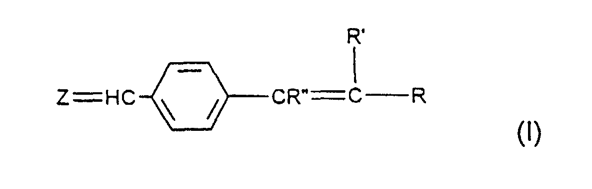 où R, R' et Z ont les significations données dans la revendication 1, caractérisé en ce que R" représente un reste de formule
